# EUROPÄISCHE PATENTSCHRIFT

(11) **EP 1 254 096 B1**
(45) Veröffentlichungstag und Bekanntmachung des Hinweises auf die Patenterteilung: **09.11.2005**
(21) Anmeldenummer: 01907453.3
(22) Anmeldetag: 17.01.2001
(51) Int. Cl.: C07C 29/00, C07C 17/00, C07C 41/01, C07C 67/00, C07C 33/46, C07C 22/04, C07C 43/174, C07C 69/62

(54) **VERFAHREN ZUR HERSTELLUNG VON 2,3,5,6-TETRAHALOGEN-XYLYLIDENVERBINDUNGEN**
METHOD FOR PRODUCING 2,3,5,6-TETRAHALOGEN-XYLYLIDENE COMPOUNDS
PROCEDE DE PREPARATION DE COMPOSES 2,3,5,6-TETRAHALOGENE-XYLYLIDENE

(30) Priorität: 27.01.2000 DE 10003320
(43) Veröffentlichungstag der Anmeldung: 06.11.2002
(73) Patentinhaber: LANXESS Deutschland GmbH, 51369 Leverkusen (DE)
(72) Erfinder: LANGER, Reinhard, 47918 Tönisvorst (DE); RODEFELD, Lars, 51371 Leverkusen (DE)
(86) Internationale Anmeldenummer: PCT/EP2001/000446
(87) Internationale Veröffentlichungsnummer: WO 2001/055064

(56) Entgegenhaltungen:
- EP-A- 0 099 622
- EP-A- 0 152 174
- GB-A- 2 127 013
- DATABASE CHEMABS [Online] CHEMICAL ABSTRACTS SERVICE, COLUMBUS, OHIO, US; SUMITOMO CHEMICAL CO., LTD., JAPAN: "Hydroxydiphenylamines" retrieved from STN Database accession no. 102:113026 CA XP002169098 & JP 59 206338 A (SUMITOMO CHEMICAL CO., LTD., JAPAN) 22. November 1984 (1984-11-22)

## Beschreibung

Die vorliegende Erfindung betrifft die Herstellung von 2,3,5,6-Tetrahalogen-xylylidenverbindungen durch Diazotierung von 2,3,5,6-Tetrahalogen-xylylidendiamin.

2,3,5,6-Tetrahalogen-xylylidenverbindungen sind dem Fachmann als wichtige Zwischenprodukte bekannt, beispielsweise zur Herstellung von pharmazeutischen oder agrochemischen Wirkstoffen wie Insektiziden.

Zur Herstellung von 2,3,5,6-Tetahalogen-xylylidendiolen, deren Estern und Ethem sind nur wenige Verfahrensweisen Stand der Technik: In der GB-A-21 27 013 wird beispielsweise die Reduktion von Tetrafluorterephthalylsäurechlorid mit Natriumborhydrid zum Tetrafluorxylylidendiol beschrieben sowie die nachfolgende Veresterung mit Acetylchlorid zum Tetrafluorterephthalylacetat.

Aus EP-A-0 152 174 ist ferner eine komplizierte Reaktionsfolge bekannt, bei der man ausgehend von 2,3,5,6-Tetrafluortoluol durch Metallierung, Methylierung, Bromierung, Substitution des Xylylidendibromides mit Kaliumacetat und Hydrolyse zum 2,3,5,6-Tetrahalogen-xylylidendiol gelangt.

Die Herstellung von Tetrahalogen-xylylidendiamin durch Hydrierung von Tetrahalogenterephthalonitril mit einem Hydrierkatalysator auf der Basis von Rhodium, Palladium, Ruthenium, Nickel, Kobalt oder Platin sowie in Gegenwart von Säuren wird in EP-A-099 622 beschrieben.

Die Umsetzung von 2,3,5,6-Tetrahalogen-xylylidendiamin zu 2,3,5,6-Tetrahalogen-xylylidenverbindungen unter Spaltung der N-C-Bindung ist bisher nicht beschrieben worden.

Die Aufgabe der vorliegenden Erfindung bestand darin, ein Verfahren bereitzustellen, welches eine technische Herstellung von 2,3,5,6-Tetrahalogen-xylylidenverbindungen mit hohen Raum-Zeit-Ausbeuten aus einfachen Vorstufen ermöglicht.

Gegenstand der Erfindung ist ein Verfahren zur Herstellung von 2,3,5,6-Tetrahalogen-xylylidenverbindungen der allgemeinen Formel (I) 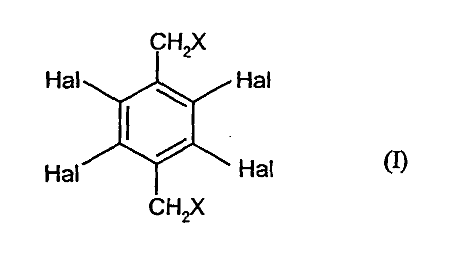 wobei Hal gleich oder verschieden ist und für Fluor oder Chlor und X für Fluor, Chlor, Brom, -O-R oder -O-C(=O)R steht, wobei R Wasserstoff, einen geradkettigen oder verzweigten C₁-C₁₂-Alkyl- oder einen C₆-C₁₄-Aryl-Rest darstellt,
durch Umsetzung von 2,3,5,6-Tetrahalogen-xylylidendiaminen der allgemeinen Formel (II) 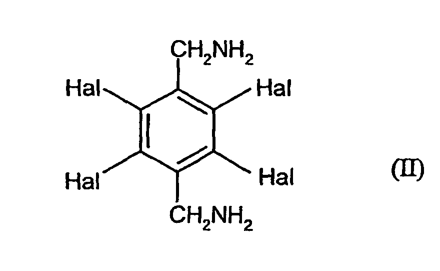 wobei Hal die für die allgemeine Formel (I) genannte Bedeutung hat,
dadurch gekennzeichnet, dass Verbindungen der allgemeinen Formel (II) mit Alkylnitriten und/oder salpetriger Säure in HX als Lösungsmittel umgesetzt werden, wobei X in HX die für die allgemeine Formel (I) genannte Bedeutung hat.

Im erfindungsgemäßen Verfahren steht der Substituent X in der allgemeinen Formel (I) sowie in HX bevorzugt für Fluor, Hydroxy, OR oder -O-C(=O)R, wobei R für Wasserstoff, einen geradkettigen oder verzweigten C₁-C₆, insbesondere C₁-C₄-Alkylrest steht. Besonders bevorzugt wird als Lösungsmittel HX Wasser verwendet, so dass als Verbindungen der allgemeinen Formel (I) 2,3,5,6-Tetrahalogen-xylylidendiole erhalten werden.

Die Diazotierung der 2,3,5,6-Tetrahalogen-xylylidendiaminen der Formel (II) erfolgt im erfindungsgemäßen Verfahren in Gegenwart von Alkylnitriten und/oder salpetriger Säure. Als Alkylnitrite haben sich C₁-C₁₀-Alkylnitrite bewährt, bevorzugt wird Isoamyl-, Ethyl- oder Methylnitrit, besonders bevorzugt Methylnitrit verwendet. Anstelle oder zusätzlich zu dem Alkylnitrit kann auch salpetrige Säure verwendet werden. Diese kann auch in-situ aus Alkali- und/oder Erdalkalinitrit und einer Mineralsäure und/oder Carbonsäure hergestellt werden. In diesem Fall werden als Alkali- und/oder Erdalkalinitrit bevorzugt Calcium-, Magnesium-, Natrium- oder Kaliumnitrit eingesetzt und als Mineralsäure bevorzugt Schwefel-, Salz-, Fluss-, Chlorsulfon-, Fluorsulfon- oder Phosphorsäure. Als Carbonsäure kann beispielsweise Ameisensäure, Essigsäure, Propionsäure oder Benzoesäure verwendet werden.

In einer bevorzugten Ausführungsform des erfindungsgemäßen Verfahrens werden neben dem Lösungsmittel HX noch ein oder mehrere andere inerte Lösungsmittel verwendet. Als andere inerte Lösungsmittel sind für das erfindungsgemäße Verfahren C₃-C₃₀-, bevorzugt C₆-C₁₂-Alkane, C₅-C₃₀-, bevorzugt C₆-C₁₂-Cycloalkane, C₆-C₂₀-, bevorzugt C₆-C₁₈-Aromaten, C₃-C₃₀-Ester und C₃-C₃₀-Ether geeignet. Bevorzugt werden Hexan, Heptan, Petrolether, Cyclohexan, Decalin, Benzol, Toluol, die Xylole, Chlorbenzol, Dichlorbenzol, Trichlorbenzol, Essigester, Butylacetat, Diethylether, Tetrahydrofuran oder Diphenylether eingesetzt.

Es hat sich ferner bewährt, den pH-Wert des Reaktionsgemisches durch Zusatz von Mineralsäuren und/oder Carbonsäuren auf einen Wert von 0 - 9, bevorzugt von 2 - 7 und besonders bevorzugt von 3 - 5 einzustellen. Als Mineral- bzw. Carbonsäuren können dabei die bereits oben für die in-situ-Herstellung der salpetrigen Säure erwähnten Verbindungen zum Einsatz kommen.

Das erfindungsgemäße Verfahren wird bei Temperaturen von 0 - 100°C, bevorzugt von 30 - 60°C durchgeführt.

Der Druck liegt während der Diazotierung bei 0,2 - 200 bar, bevorzugt bei 0,5 - 50 bar und besonders bevorzugt bei 1 - 10 bar.

Das Molverhältnis der Verbindungen der Formel (II) zum Alkylnitrit und/oder zur salpetrigen Säure beträgt 1: (2-10), bevorzugt 1: (2-4). Die Reaktionslösung besitzt einen Eduktgehalt von 1-50 Gew.%, bevorzugt 5-25 Gew.%.

Das erfindungsgemäße Verfahren kann in üblichen Reaktoren diskontinuierlich, semikontinuierlich oder kontinuierlich durchgeführt werden. Die exakte technische Ausgestaltung hängt von den Verfahrensparametern ab und ist für den Fachmann leicht zu ermitteln.

Ausgangsstoffe für das erfindungsgemäße Verfahren sind Verbindungen der Formel (II), die bevorzugt durch Hydrierung der entsprechenden Tetrahalogenterephthalonitrile gewonnen werden.

In einer bevorzugten Ausführungsform wird das erfindungsgemäße Verfahren daher so durchgeführt, dass die Verbindungen der Formel (II) durch Hydrierung von Verbindungen der allgemeinen Formel (III) 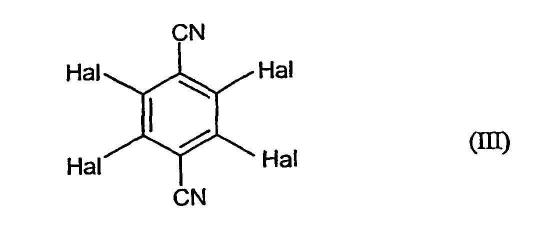 wobei Hal und X die für die allgemeine Formel (I) genannten Bedeutungen besitzen;
in Gegenwart eines Hydrierkatalysators und eines Lösungsmittels HX hergestellt werden.

Als Hydrierkatalysatoren können alle für die Hydrierung von Nitrilen üblichen und bekannten Katalysatoren eingesetzt werden. Bewährt haben sich vor allem Katalysatoren auf der Basis von Elementen der VIII-Nebengruppe. Bevorzugt werden Hydrierkatalysatoren auf der Basis von Rhodium, Palladium, Ruthenium, Nickel, Kobalt oder Platin eingesetzt, besonders bevorzugt sind Palladium-Kontakte.

Die Hydrierung wird ferner in Gegenwart von einem oder mehreren Lösungsmitteln HX durchgeführt, wobei X die für die Formel (I) genannten Bedeutungen hat. Gegebenenfalls können auch weitere inerte Lösungsmittel zugegen sein. Die Reaktionstemperatur der Hydrierung liegt bei Temperaturen von -30 bis +60°C, bevorzugt bei 0 bis 30°C und der Druck beträgt 3 - 300 bar, bevorzugt 10 - 100 bar. Bevorzugt wird in Gegenwart von Mineralsäuren und/oder Carbonsäuren hydriert. Als Mineral- und Carbonsäuren können dabei die bereits für das erfindungsgemäße Verfahren genannten Säuren verwendet werden.

Besonders vorteilhaft ist eine Verfahrensvariante, die dadurch gekennzeichnet ist, dass die durch Hydrierung gewonnene Verbindung der Formel (II) von dem Hydrierkatalysator durch Filtration befreit wird und gegebenenfalls nach destillativer Abtrennung störenden Lösungsmittels der Diazotierung direkt ohne Zwischenisolierung zugeführt wird. Dabei kann diese nach der Hydrierung vorliegende Reaktionslösung vorgelegt und dann das Diazotierungsreagzenz Alkylnitrit und/oder salpetrige Säure zugegeben werden, es kann jedoch auch umgekehrt verfahren werden.

Das erfindungsgemäße Verfahren liefert über einfache Verfahrensschritte die gewünschten 2,3,5,6-Tetrahalo-xylylidenverbindungen in hoher Reinheit und mit hoher Raum-Zeit-Ausbeute.

### Beispiele

### Beispiel 1

### Herstellung des 2,3,5,6-Tetrafluor-xylylidendiamins als schwefelsaure Lösung:

1249,5 g 2,3,5,6-Tetrafluorterephthalsäuredinitril (6,24 mol) werden zusammen mit 750 ml Methanol, 5250 ml Wasser, 1050 g konzentrierter Schwefelsäure und 75 g eines 5%igen Palladium-Kohle Katalysators in einem10 1 Autoklav vorgelegt und bei 30°C und 30 bar Wasserstoffdruck bis zur Druckkonstanz hydriert. Der Katalysator wird abfiltriert. Anschließend wird das Methanol bei 80-90°C und 350 mbar wieder abdestilliert. Man erhält 6690 g einer schwefelsauren Lösung, die nach GC Analyse (externer Standard) 1197,2 g (5,75 mol) Tetrafluor-xylylidendiamin (92 % Ausbeute) enthält.

### Beispiel 2

### Herstellung des 2,3,5,6-Tetrafluorxylylidendiols:

Von einer wie oben hergestellten Lösung Tetrafluor-xylylidendiamin Hydrosulfat werden 3200 g (2,87 mol Verbindung) mit 868 g 20%iger Natriumhydroxid-Lösung auf den pH 4 eingestellt. Die Reaktionsmischung wird auf 80-90°C erhitzt und lässt innerhalb von 4 h 981 g einer 40%igen Natriumnitrit-Lösung zutropfen. Gleichzeitig hält man den pH-Wert der Lösung mit Hilfe von 127 g 30%iger Schwefelsäure zwischen pH 3 und pH 5. Nach beendeter Gasentwicklung wird der Reaktionsansatz zweimal mit 750 ml Ethylacetat extrahiert und die organische Phase anschließend im Vakuum eingeengt. Man erhält 456 g (2,17 mol) 2,3,5,6-Tetrafluorxylylidendiol in einer Reinheit von 74,5 % und einer Ausbeute von 76 %.

## Patentansprüche

1. Verfahren zur Herstellung von 2,3,5,6-Tetrahalogen-xylylidenverbindungen der allgemeinen Formel (I) 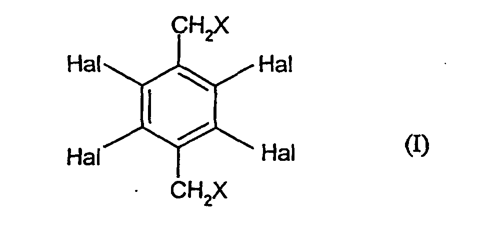 wobei Hal gleich oder verschieden ist und für Fluor oder Chlor und X für Fluor, Chlor, Brom, -O-R oder -O-C(=O)R steht, wobei R Wasserstoff, einen geradkettigen oder verzweigten C₁-C₁₂-Alkyl- oder einen C₆-C₁₄-Aryl-Rest darstellt,
durch Umsetzung von 2,3,5,6-Tetrahalogen-xylylidendiaminen der allgemeinen Formel (II) 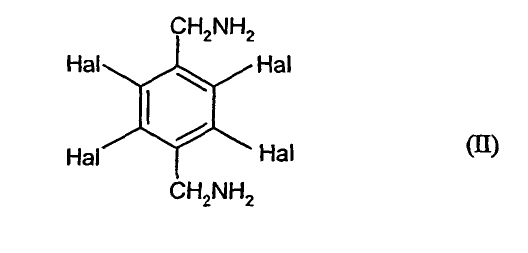 wobei Hal die für die allgemeine Formel (I) genannte Bedeutung hat,
**dadurch gekennzeichnet, dass** Verbindungen der allgemeinen Formel (II) mit Alkylnitriten und/oder salpetriger Säure in HX als Lösungsmittel umgesetzt werden, wobei X die für die allgemeine Formel (I) genannte Bedeutung hat.

2. Verfahren nach Anspruch 1, **dadurch gekennzeichnet, dass** X in der allgemeinen Formel (I) bzw. HX für Fluor, Hydroxy, OR oder -O-C(=O)R steht, wobei R für Wasserstoff oder C₁-C₆-, bevorzugt C₁-C₄-Alkyl steht.

3. Verfahren nach Anspruch 1 oder 2, **dadurch gekennzeichnet, dass** als Alkylnitrite C₁-C₁₀-Alkylnitrite, bevorzugt Isoamyl-, Ethyl- oder Methylnitrit eingesetzt werden.

4. Verfahren nach einem oder mehreren der Ansprüche 1-3, **dadurch gekennzeichnet, dass** anstelle oder zusätzlich zu dem Alkylnitrit salpetrige Säure verwendet wird.

5. Verfahren nach Anspruch 4, **dadurch gekennzeichnet, dass** die salpetrige Säure in-situ aus Alkali- und/oder Erdalkalinitrit und einer Mineralsäure und/oder Carbonsäure hergestellt wird.

6. Verfahren nach Anspruch 5, **dadurch gekennzeichnet, dass** als Alkali- und/oder Erdalkalinitrit Calcium-, Magnesium-, Natrium- oder Kaliumnitrit, als Mineralsäure Schwefel-, Salz-, Fluss-, Chlorsulfon-, Fluorsulfon- oder Phosphorsäure und/oder als Carbonsäure Ameisensäure, Essigsäure, Propionsäure oder Benzoesäure verwendet werden.

7. Verfahren nach einem oder mehreren der Ansprüche 1-6, **dadurch gekennzeichnet, dass** in Gegenwart von Wasser als Lösungsmittel HX gearbeitet wird.

8. Verfahren nach einem oder mehreren der Ansprüche 1-7, **dadurch gekennzeichnet, dass** der pH-Wert des Reaktionsgemisches durch Zusatz der in Anspruch 6 genannten Mineralsäuren und/oder Carbonsäuren auf einen Wert von 0 - 9, bevorzugt von 2 - 7 und besonders bevorzugt von 3 - 5 eingestellt wird.

9. Verfahren nach einem oder mehreren der Ansprüche 1-8, **dadurch gekennzeichnet, dass** bei Temperaturen von 0 - 100°C, bevorzugt von 30 - 60°C und einem Druck von 0,2 - 200 bar, bevorzugt von 0,5 - 50 bar und besonders bevorzugt von 1 - 10 bar gearbeitet wird.

10. Verfahren nach einem oder mehreren der Ansprüche 1-9, wobei die Verbindungen der allgemeinen Formel (II) durch Hydrierung von Verbindungen der allgemeinen Formel (III) in Gegenwart eines Hydrierkatalysators und eines Lösungsmittels HX, 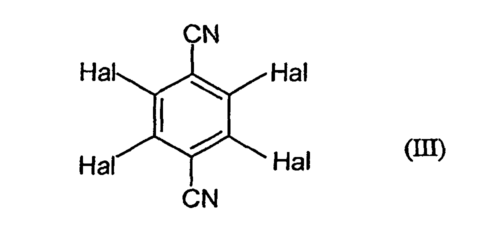 wobei Hal und X die für die allgemeine Formel (I) genannten Bedeutungen besitzen, erhalten werden.

11. Verfahren nach Anspruch 10, **dadurch gekennzeichnet, dass** Hydrierkatalysatoren auf der Basis von Elementen der VIII-Nebengruppe, bevorzugt auf der Basis von Rhodium, Palladium, Ruthenium, Nickel, Kobalt oder Platin eingesetzt werden.

12. Verfahren nach Anspruch 10 oder 11, **dadurch gekennzeichnet, dass** die Hydrierung in Gegenwart von einem oder mehreren Lösungsmittel HX durchgeführt wird, wobei X die für die Formel (I) genannten Bedeutungen hat, die Reaktionstemperatur der Hydrierung bei -30 bis +60°C, bevorzugt bei 0 bis 30°C und der Druck bei 3 - 300 bar, bevorzugt 10 - 100 bar liegt.

13. Verfahren nach einem oder mehreren der Ansprüche 10-12, **dadurch gekennzeichnet, dass** die durch Hydrierung gewonnene Verbindung der Formel (II) durch Filtration von dem Hydrierkatalysator befreit wird und gegebenenfalls nach Abtrennung des Lösungsmittels durch Destillation direkt dem Verfahren nach einem oder mehreren der Ansprüche 1-8 zugeführt wird.

## Claims

1. Process for preparing 2,3,5,6-tetrahaloxylylidene compounds of the general formula (I) 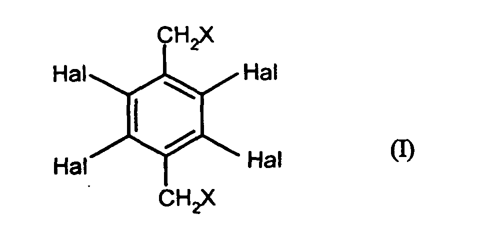 where each Hal, which may be the same or different, is fluorine or chlorine and X is fluorine, chlorine, bromine, -O-R or -O-C(=O)R, where R is hydrogen, a straight-chain or branched C₁-C₁₂-alkyl- or a C₆-C₁₄-aryl radical,
by reacting 2,3,5,6-tetrahaloxylylidenediamines of the general formula (II) 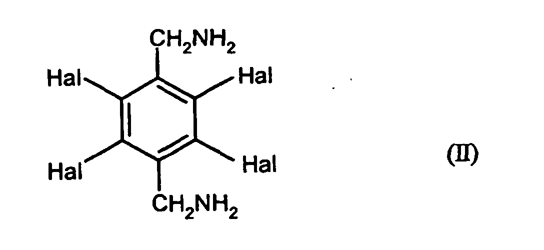 where Hal is as defined for the general formula (I),
**characterized in that** compounds of the general formula (II) are reacted with alkyl nitrites and/or nitrous acid in HX as a solvent, where X is as defined for the general formula (I).

2. Process according to Claim 1, **characterized in that** X in the general formula (I) or HX is fluorine, hydroxyl, OR or -O-C(=O)R, where R is hydrogen or C₁-C₆-alkyl, and preferably C₁-C₄-alkyl.

3. Process according to Claim 1 or 2, **characterized in that** the alkyl nitrites used are C₁-C₁₀-alkyl nitrites, preferably isoamyl, ethyl or methyl nitrite.

4. Process according to one or more of Claims 1 to 3, **characterized in that**, instead of or in addition to the alkyl nitrite, nitrous acid is used.

5. Process according to Claim 4, **characterized in that** the nitrous acid is prepared in situ from an alkali metal nitrite and/or an alkaline earth metal nitrite and a mineral acid and/or a carboxylic acid.

6. Process according to Claim 5, **characterized in that** the alkali metal nitrite or alkaline earth metal nitrite used is calcium nitrite, magnesium nitrite, sodium nitrite or potassium nitrite, and the mineral acid used is sulphuric acid, hydrochloric acid, hydrofluoric acid, chlorosulphonic acid, fluorosulphonic acid or phosphoric acid and/or the carboxylic acid used is formic acid, acetic acid, propionic acid or benzoic acid.

7. Process according to one or more of Claims 1 to 6, **characterized in that** operation is effected in the presence of water using HX as solvent.

8. Process according to one or more of Claims 1 to 7, **characterized in that** the pH of the reaction mixture is adjusted by addition of the mineral acids and/or carboxylic acids mentioned in Claim 6 to a value of from 0 to 9, preferably from 2 to 7 and more preferably from 3 to 5.

9. Process according to one or more of Claims 1 to 8, **characterized in that** operation is effected at temperatures of from 0 to 100°C, preferably from 30 to 60°C, and a pressure of from 0.2 to 200 bar, preferably from 0.5 to 50 bar and more preferably from 1 to 10 bar.

10. Process according to one or more of Claims 1-9, **characterized in that** the compounds of the general formula (II) are obtained by hydrogenation of compounds of the general formula (III) in the presence of a hydrogenation catalyst and a solvent HX, 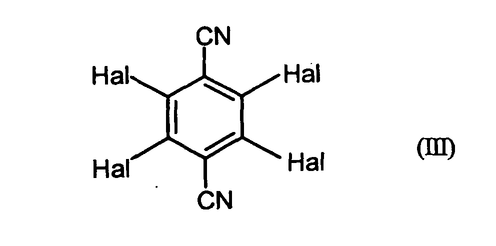 where Hal and X are as defined for the general formula (I).

11. Process according to Claim 10, **characterized in that** hydrogenation catalysts based on elements of transition group VIII of the periodic table, preferably based on rhodium, palladium, ruthenium, nickel, cobalt or platinum, are used.

12. Process according to Claim 10 or 11, **characterized in that** the hydrogenation is carried out in the presence of one or more solvents HX, where X is as defined for the formula (I), the reaction temperature of the hydrogenation is from -30 to +60°C, preferably from 0 to 30°C, and the pressure is from 3 to 300 bar, preferably from 10 to 100 bar.

13. Process according to one or more of Claims 10 to 12, **characterized in that** the compound of the formula (II) obtained by hydrogenation is freed from the hydrogenation catalyst by filtration and, if necessary after distillative removal of the solvent, is introduced directly to the process as claimed in one or more of Claims 1 to 8.

## Revendications

1. Procédé de préparation de composés de 2,3,5,6-tétrahalogénoxylylidène de formule générale (I) 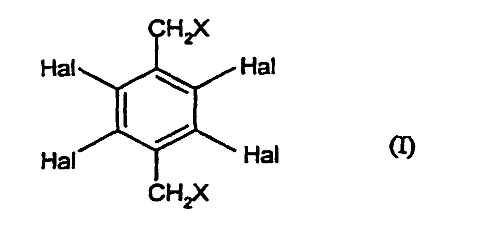 dans laquelle les Hal sont identiques ou différents et représentent le fluor ou le chlore, et X représente le fluor, le chlore, le brome, -O-R ou -O-C(=O)R, où R représente l'hydrogène ou un reste alkyle en C₁-C₁₂ linéaire ou ramifié ou aryle en C₆-C₁₄,
par réaction de 2,3,5,6-tétrahalogéno-xylylidènediamines de formule générale (II) 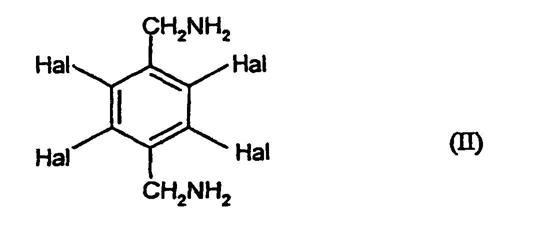 dans laquelle Hal a la signification indiquée pour la formule générale (I),
**caractérisé en ce que** l'on fait réagir des composés de formule générale (II) avec des nitrites d'alkyle et/ou de l'acide nitreux dans du HX comme solvant, X ayant la signification indiquée pour la formule générale (I).

2. Procédé selon la revendication 1, **caractérisé en ce que** X, dans la formule générale (I) ou dans HX, représente le fluor ou un groupe hydroxy ou -O-C(=O)R, où R représente l'hydrogène ou un groupe alkyle en C₁-C₆, de préférence en C₁-C₄.

3. Procédé selon la revendication 1 ou 2, **caractérisé en ce que**, comme nitrites d'alkyle, on utilise des nitrites d'alkyle en C₁-C₁₀, de préférence le nitrite d'isoamyle, d'éthyle ou de méthyle.

4. Procédé selon l'une ou plusieurs des revendications 1-3, **caractérisé en ce que** l'on utilise de l'acide nitreux à la place ou en plus du nitrite d'alkyle.

5. Procédé selon la revendication 4, **caractérisé en ce que** l'acide nitreux est préparé *in situ* à partir de nitrite de métal alcalin et/ou de métal alcalino-terreux et d'un acide inorganique et/ou d'un acide carboxylique.

6. Procédé selon la revendication 5, **caractérisé en ce que** on utilise du nitrite de calcium, de magnésium, de sodium ou de potassium comme nitrite de métal alcalin ou de métal alcalino-terreux, de l'acide sulfurique, de l'acide chlorhydrique, de l'acide fluorhydrique, de l'acide chlorosulfonique, de l'acide fluorosulfonique ou de l'acide phosphorique comme acide inorganique et/ou de l'acide formique, de l'acide acétique, de l'acide propionique ou de l'acide benzoïque comme acide carboxylique.

7. Procédé selon l'une ou plusieurs des revendications 1-6, **caractérisé en ce que** l'on travaille en présence d'eau comme solvant HX.

8. Procédé selon l'une ou plusieurs des revendications 1-7, **caractérisé en ce que** l'on ajuste le pH du mélange réactionnel à une valeur de 0-9, de préférence de 2-7, et de façon particulièrement préférée de 3-5 en ajoutant les acides inorganiques et/ou les acides carboxyliques indiqués dans la revendication 6.

9. Procédé selon l'une ou plusieurs des revendications 1-8, **caractérisé en ce que** l'on travaille à des températures de 0-100°C, de préférence de 30-60°C et sous une pression de 0,2-200 bar, de préférence de 0,5-50 bar et de façon particulièrement préférée de 1-10 bar.

10. Procédé selon l'une ou plusieurs des revendications 1-9, dans lequel les composés de formule générale (II) sont obtenus par hydrogénation de composés de formule générale (III) en présence d'un catalyseur d'hydrogénation et d'un solvant HX 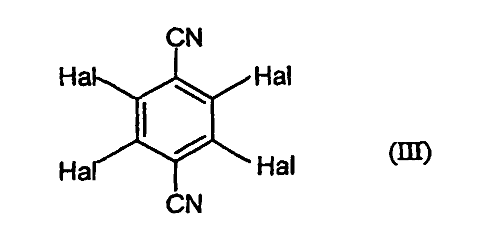 où Hal et X ont les significations indiquées pour la formule générale (I).

11. Procédé selon la revendication 10, **caractérisé en ce que** l'on utilise des catalyseurs d'hydrogénation à base d'éléments du groupe secondaire VIII, de préférence à base de rhodium, de palladium, de ruthénium, de nickel, de cobalt ou de platine.

12. Procédé selon la revendication 10 ou 11, **caractérisé en ce que** l'hydrogénation s'effectue en présence d'un ou de plusieurs solvants HX, X ayant la signification indiquée pour la formule (I), la température de la réaction d'hydrogénation est de -30 à +60°C, de préférence de 0 à 30°C, et la pression est de 3-300 bar, de préférence de 10-100 bar.

13. Procédé selon l'une ou plusieurs des revendications 10-12, **caractérisé en ce que** l'on sépare par filtration le catalyseur d'hydrogénation du composé de formule (II) obtenu par l'hydrogénation, que l'on envoie directement dans le procédé selon l'une ou plusieurs des revendications 1-8, éventuellement après avoir séparé le solvant par distillation.
